# EUROPEAN PATENT SPECIFICATION

(11) **EP 2 706 994 B1**
(45) Date of publication and mention of the grant of the patent: **01.03.2017**
(21) Application number: 12731678.4
(22) Date of filing: 10.05.2012
(51) Int. Cl.: A61K 9/00, A61K 9/16, A61K 33/38

(54) **PHARMACEUTICAL TOPICAL COMPOSITION FOR USE IN THE TREATMENT AND/OR IN THE PREVENTION OF INFECTIONS OF SKIN LESIONS**
PHARMAZEUTISCHE TOPISCHE ZUSAMMENSETZUNG ZUR VERWENDUNG BEI DER BEHANDLUNG UND/ODER PRÄVENTION VON INFEKTIONEN IM FALL VON HAUTLÄSIONEN
COMPOSITION PHARMACEUTIQUE TOPIQUE DESTINÉE À ÊTRE UTILISÉE DANS LE TRAITEMENT ET/OU DANS LA PRÉVENTION D'INFECTIONS DE LÉSIONS CUTANÉES

(30) Priority: 11.05.2011 IT MI20110818
(43) Date of publication of application: 19.03.2014
(73) Proprietor: Soveta Baltica Uab, 08221 Vilnius (LT)
(72) Inventor: LABRUZZO, Carla, I-20154 Milano (IT)
(74) Representative: Pistolesi, Roberto
(86) International application number: PCT/IB2012/052324
(87) International publication number: WO 2012/153288

(56) References cited:
- EP-A2- 2 123 309
- WO-A2-2008/051513
- DE-A1- 2 804 931
- LEE JIN-CHUANN ET AL: "Therapeutic effect of two mineral drugs on thermal skin injury in rabbits", ZHONGHUA YAOXUE ZAZHI - CHINESE PHARMACEUTICAL JOURNAL, ZHONGGUO YAOXUEHUI, TAIPEI, TW, vol. 50, no. 1, 1 February 1998 (1998-02-01), pages 25-32, XP009089767, ISSN: 1016-1015 cited in the application
- LINARES H A ET AL: "EVALUATION OF TOPICAL THERAPY WITH SILVER-KAOLIN ARGOSTOP IN AN EXPERIMENTAL MODEL OF BURN WOUND SEPSIS", BURNS, vol. 13, no. 4, 1987, pages 281-285, XP002666965, ISSN: 0305-4179 cited in the application

## Description

The present invention relates to a topical composition in powder spray form containing silver sulfadiazine and at least one silicate, for use in the treatment and/or prevention of infections of skin lesions.

### STATE OF THE ART

Any alteration in the skin surface caused by a pathological, traumatic, or environmental event is defined 'skin lesion'. The most severe skin lesions, usually associated with burns, are classified according to the degree of damage caused to the tissue. The main complication from burns and serious lesions is represented by the infections caused by the fact that the lesioned and/or burnt skin is prone to colonisation by bacteria, usually from the patient skin bacterial flora. One of the local treatments for infections in the event of first- and second-degree burns is silver sulfadiazine.

Silver sulfadiazine is the international non-proprietary name (INN) of the active ingredient silver [(4-aminophenyl)sulfonyl](pyrimidin-2-yl)azanide, whose structural formula is given below.

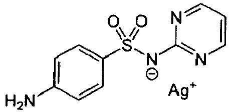

This compound is also used in local treatment of infected bedsores and varicose ulcers or, in general, of dermatological affections in the event of infection or susceptibility to superinfections.

Several bibliographic documents exist documenting its effectiveness in the aforesaid treatment of infections of burn wounds.

U.S. patent 4,551,139 describes an apparatus and a method for the spray application of a cream containing silver sulfadiazine to a burn wound. The aforesaid cream is preferably sprayed onto sterile gauze prior to application.

Foroutan et al. (Iranian Journal of Pharmaceutical Research, 2002, 1, 47-49) describes a spray formulation containing sulfadiazine silver in double-distilled water. The effectiveness of the aforementioned spray formulation was evaluated *in vitro* on the *Pseudomonas aeroginosa* strain and compared with the corresponding cream formulation, demonstrating similar antimicrobial activity.

U.S. patent 6,987,133 describes a topical preparation in spray form containing silver sulfadiazine, dispersed or solubilised in a cream or lotion, for the treatment of microbial infections and burns.

Linares et al. (Burns, 1987, 13, 281-285) describes a clinical trial performed on rats, to evaluate the effectiveness of a silver-kaolin composition in the treatment of sepsis deriving from burn wounds. This composition contains 5% metallic silver particles dispersed in kaolin. The resulting composition was shown to have an antimicrobial activity comparable with the silver sulfadiazine alone for both prevention and treatment of bacterial infections.

Patent application WO2008/051513 describes the use of topical compositions comprising silicon dioxide particles, in combination with an anti-infective drug, such as, for example, silver sulfadiazine, to reduce bleeding or exudation in skin wounds. European patent application EP2123309 describes the use of a nano-silica or -silicate coating applied to diverse surfaces. Furthermore, the haemostatic product thereby obtained may include one or more therapeutic agents, among which silver sulfadiazine is mentioned.

Lee et al. (Chinese Pharmaceutical Journal 1998, 50, 25-32) describes a comparative study on the therapeutic effects of talc, *kaolinum* (identified as a mixture of hydrated halloysite and kaolinite), kaolin, and 1% silver sulfadiazine applied to burns and cut wounds. This study highlights the increased effectiveness of *kaolinum* in minimising scar formation, while kaolin, as a purified form of *kaolinum*, is not so effective.

The patent application DE2804931 relates to a method of preparation of silver and zinc sulfadiazine, formulated in a topical form.

However, a cream formulation of a composition containing silver sulfadiazine poses several problems, such as, for example, those relating to its application since, in order to be effective, the cream must be spread in a layer measuring a few millimetres, using a sterile glove or a gauze. Furthermore, some time after spreading, the surface area covered by the cream tends to decrease and consequently the wound edges are no longer covered by the cream.

Therefore there is felt the need for an alternative formulation which allows both an easy application of the active ingredient to skin lesions and a wound coverage which remains stable over time.

### BRIEF DESCRIPTION OF THE FIGURES

Figure 1: Particle size profile of the end product (Batch 1)
Figure 2: Particle size profile of the end product (Batch 2)

### DESCRIPTION

It has been surprisingly found that a topical composition in powder spray form containing silver sulfadiazine and at least one silicate can be used in the treatment and/or prevention of infection of skin lesions.

The present invention relates to a composition in powder spray form containing silver sulfadiazine and at least one silicate, and preferably, at least one pharmaceutically acceptable excipient, for use in the treatment and/or prevention of infection of skin lesions, such as abrasions, excoriations, cuts, superficial wounds, and dermatological affections.

A silicate according to the present invention is preferably selected from hydrated magnesium silicate, calcium silicate, aluminium silicate, or a mixture thereof.

A particularly preferred silicate according to the present invention is an aluminium silicate.

An aluminium silicate according to the present invention is preferably kaolin, and more preferably light kaolin.

According to the *British Pharmacopoeia 2011 Edition*, "light kaolin" refers to a natural hydrated aluminium silicate, which has been purified by elutriation and dried. Preferred excipients according to the present invention are selected from calcium gluconate and/or colloidal silica.

It has now been surprisingly found that the said silicate creates a mechanical barrier against external agents, protecting damaged skin. The presence of said silicate in the composition according to the present invention, also allows exudates formed during the inflammatory processes of the skin lesion to be drained and absorbed.

Thanks to the powder spray form, it is now possible to distribute the antibacterial agent, such as, the silver sulfadiazine, in a uniform manner over the area affected by the skin wound, unlike with a different topical formulation in powder form. In addition, such an agent within the composition of the present invention not only allows to inhibit the growth of microorganisms in said area, but also prevents contamination of the wound by external agents.

The results obtained stated below in the experimental part demonstrate an effectiveness of the aforesaid composition that is very similar to the cream form, already known in the field, without, however, presenting the typical drawbacks thereof discussed earlier.

According to the present invention, the composition contains a quantity of silver sulfadiazine comprised between 0.1% and 5% by weight, preferably between 0.5% and 2% by weight, with respect to the total weight of the composition.

Furthermore, according to the present invention, the aforesaid composition contains a quantity of at least one silicate comprised between 92% and 99% by weight, preferably between 94% and 98% by weight, with respect to the total weight of the composition.

A further object of the present invention is a topical composition formulated in powder form. The said powder form is preferably in spray form or as dispersion powder, more preferably in spray form.

The composition according to the present invention is therefore applied directly to the patient skin lesion as a powder.

A further object of the present invention is, therefore, a topical composition in the form of powder spray which does not contain water and/or organic solvents.

This absence of water or organic solvents guarantees application and coverage of the lesioned skin which is more stable over time.

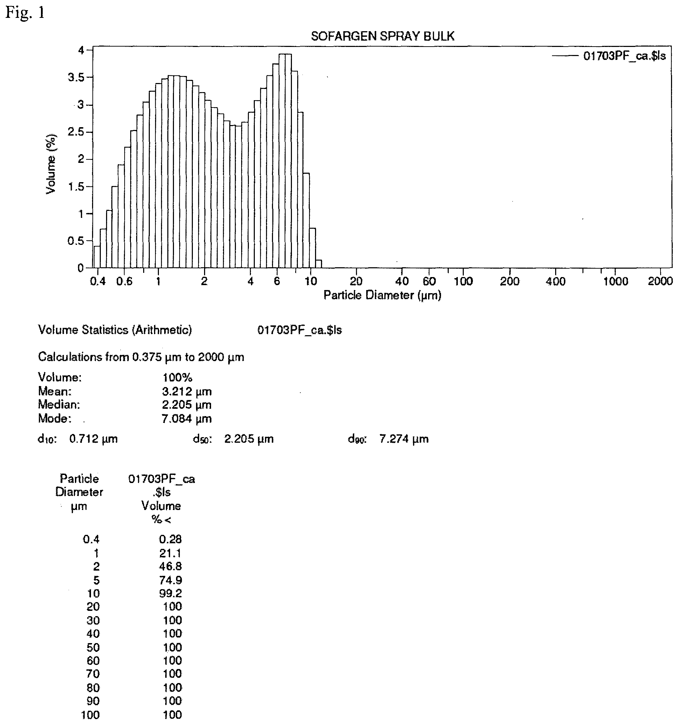

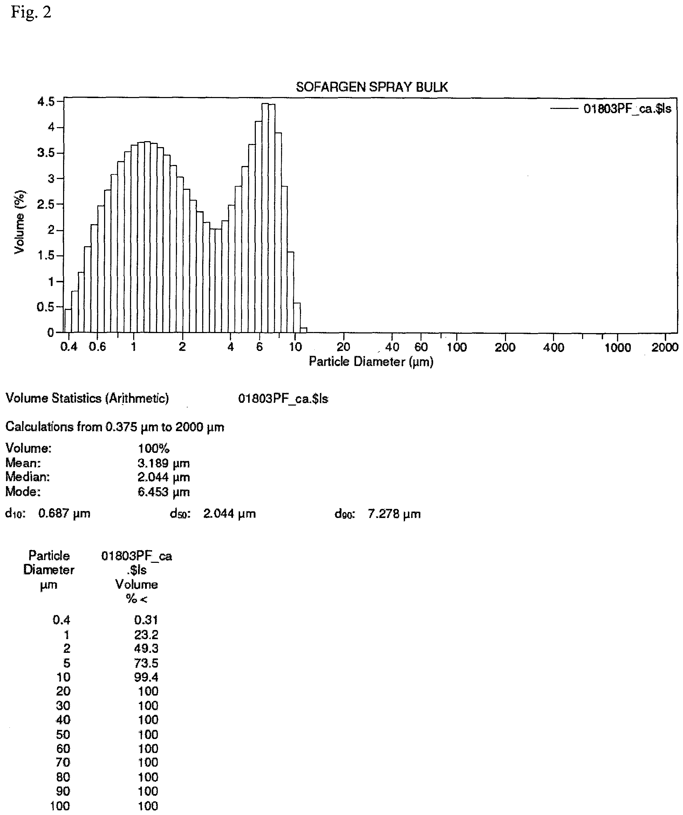

A further object of the present invention is the particle size profile of said silver sulfadiazine, said at least one silicate, and of said at least one pharmaceutically acceptable excipient, having a particle size less than 13 µm, and/or 90% of the particles having a size comprised between 5 µm and 10 µm and/or 50% of the particles having a size less than 4 µm, as shown in Figures 1 and 2.

This particle size allows to increase the surface area of contact between said silver sulfadiazine and at least one silicate and the part of damaged skin and consequently to amplify the effectiveness of the aforesaid composition.

In one embodiment of the present invention, said silver sulfadiazine, said at least one silicate and said at least one pharmaceutically acceptable excipient, are in micronized form.

In a further embodiment of the present invention, said silver sulfadiazine, said at least one silicate and said at least one pharmaceutically acceptable excipient are micronized separately prior to mixing, so as to optimise uniformity of the mixture.

A further object of the present invention is a topical composition in powder spray form containing a quantity of silver sulfadiazine comprised between 0.1% and 5% by weight with respect to the total weight of the composition, at least one silicate in a quantity comprised between 92% and 99% by weight with respect to the total weight of the composition and/or a pharmaceutically acceptable excipient, for use in the treatment and/or prevention of infections of skin lesions.

According to the invention, the composition of the present invention can be administered to humans, intending thereby both adults and "paediatric population", where the term "paediatric population" refers to the part of the population ranging from birth to eighteen years of age.

According to an embodiment of the present invention, said composition is administered from once to three times a day, preferably twice a day.

### EXPERIMENTAL PART

Two compositions were prepared containing silver sulfadiazine as an active ingredient.

### EXAMPLE 1

| | |
|---|---|
| Silver sulfadiazine | 1% |
| Light kaolin | 98% |
| Calcium gluconate | 1% |

### EXAMPLE 2

| | |
|---|---|
| Silver sulfadiazine | 1.15% |
| Light kaolin | 95.85% |
| Calcium gluconate | 1% |
| Aerosil R792 | 2% |

### CLINICAL TRIAL

A comparative trial has been performed on two formulations for topical use containing 1% silver sulfadiazine used in sterilisation (as conventionally accepted as the absence of pathogens) or in the reduction of the bacterial load of a loss of substance in the event of infected skin or in the prevention of infection of a non-infected loss of substance

The lesion was assessed objectively by evaluating the diameter and the extent of the perilesional erythema and the maceration, with an examination of the symptomatology, extent, and any clinical characteristics of the exudate.

The symptomatological examination of the lesion consisted in measuring the presence and intensity of signs of inflammation using a semi-quantitative ordinal scale, where 0 = absent, 1 = slight, 2 = moderate, 3 = severe.

Similarly, the entity was evaluated (0 = absent, 1 = minimal, 2 = large amount) as was the clinical features of any exudate (1 = serous, 2 = purulent serum, 3 = purulent). 35 adult patients of both sexes presenting lesions with traumatic or degenerative loss of substance were treated with 1% silver sulfadiazine spray formulation, corresponding to the composition in Example 1, and the same number with 1% silver sulfadiazine cream formulation, applied daily, after cleansing the wound with saline solution and appropriate medication, for 7 ± 2 days.

The results which emerged showed that the proportion of patients whose treatment, following examination of microbiological data, was deemed successful was the same among both the patients who were treated with cream and those treated with spray (94%). Application of the Blackwelder test to these proportions leads us to conclude the non-inferiority of the spray formulation with respect to the cream formulation (p-value = 0.0002). The lower confidence limit is lower than the maximum acceptable difference between the two treatments (-9%). For the majority of cases, in both groups, the treatment resulted in the prevention of infection (80% with the spray and 77% with the cream) and sterilisation (as conventionally accepted as the absence of pathogens) of the wound (49% with the spray and 34% with the cream).

It is therefore concluded that the effectiveness of silver sulfadiazine in the treatment of skin lesions with loss of substance is very high, and that the spray formulation is equally effective as the cream formulation both in preventing infection of the lesions and in sterilising infected lesions.

Tolerability proved excellent, generally and locally, in both treatment groups.

## Claims

1. A topical composition in powder spray form, containing silver sulfadiazine and at least one silicate, **characterised in that** said composition contains a quantity of silver sulfadiazine comprised between 0.1% and 5% by weight with respect to the total weight of the composition and a quantity of at least one silicate comprised between 92% and 99% by weight with respect to the total weight of the composition.

2. A composition, according to claim 1, **characterised in that** said composition contains a quantity of silver sulfadiazine comprised between 0.5% and 2% by weight with respect to the total weight of the composition.

3. A composition, according to any one of claims 1-2, **characterised in that** said composition contains a quantity of at least one silicate comprised between 94% and 98% by weight of the total weight of the composition.

4. A composition according to any one of the preceding claims, **characterised in that** said silicate is selected from hydrated magnesium silicate, aluminium silicate, calcium silicate, or a mixture thereof, preferably aluminium silicate.

5. A composition according to claim 3, **characterised in that** said aluminium silicate is kaolin, preferably light kaolin.

6. A composition according to any one of claims 1 - 3, **characterised in that** said composition contains at least one pharmaceutically acceptable excipient.

7. A composition, according to claim 6, **characterised in that** said at least one excipient is selected from calcium gluconate and/or colloidal silica.

8. A composition, according to any one of claims 1 - 7, **characterised in that** said silver sulfadiazine, said at least one silicate and/or said at least one pharmaceutically acceptable excipient are in micronized form.

9. A composition according to claim 8, **characterised in that** said silver sulfadiazine, said at least one silicate, and/or said at least one pharmaceutically acceptable excipient, present a particle size less than 13 µm, and/or 90% of the particles have a size comprised between 5 µm and 10 µm and/or 50% of the particles have a size less than 4 µm.

10. A composition according to any one of claims 1 - 9, **characterised in that** said composition does not contains water and/or organic solvents.

11. A composition according to any one of claims 1 - 10 for use in the treatment and/or prevention of infection of skin lesions.

12. A composition according to any one of claims 1 - 11, **characterised in that** said composition is administered from once to three times a day, preferably twice a day.

## Patentansprüche

1. Topische Zusammensetzung in Pulversprühform, welche Silbersulfadiazin und mindestens ein Silikat umfasst, **dadurch gekennzeichnet, dass** die Zusammensetzung eine Menge an Silbersulfadiazin von zwischen 0,1 Gew.-% und 5 Gew.-% enthält, bezogen auf das Gesamtgewicht der Zusammensetzung, und eine Menge von mindestens einem Silikat von zwischen 92 Gew.-% und 99 Gew.-%, bezogen auf das Gesamtgewicht der Zusammensetzung.

2. Zusammensetzung nach Anspruch 1, **dadurch gekennzeichnet, dass** die Zusammensetzung eine Menge an Silbersulfadiazin von zwischen 0,5 Gew.-% und 2 Gew.-% bezogen auf das Gesamtgewicht der Zusammensetzung enthält.

3. Zusammensetzung nach einem der Ansprüche 1 - 2, **dadurch gekennzeichnet, dass** die Zusammensetzung eine Menge von mindestens einem Silikat von zwischen 94 Gew.-% und 98 Gew.-%, bezogen auf das Gesamtgewicht der Zusammensetzung, enthält.

4. Zusammensetzung nach einem der vorstehenden Ansprüche , **dadurch gekennzeichnet, dass** das Silikat ausgewählt ist unter hydratisiertem Magnesiumsilikat, Aluminiumsilikat, Calciumsilikat, oder einem Gemisch davon, vorzugsweise Aluminiumsilikat.

5. Zusammensetzung nach Anspruch 3, **dadurch gekennzeichnet, dass** das Aluminiumsilikat Kaolin ist, vorzugsweise leichtes Kaolin.

6. Zusammensetzung nach einem der Ansprüche 1 - 3, **dadurch gekennzeichnet, dass** die Zusammensetzung mindestens einen pharmazeutisch annehmbaren Exzipienten enthält.

7. Zusammensetzung nach Anspruch 6, **dadurch gekennzeichnet, dass** der mindestens eine Exzipient ausgewählt ist unter Calciumgluconat und/oder kolloidalem Siliziumdioxid.

8. Zusammensetzung nach einem der Ansprüche 1 - 7, **dadurch gekennzeichnet, dass** das Silbersulfadiazin, das mindestens eine Silikat und/oder der mindestens eine pharmazeutisch annehmbare Exzipient in mikronisierter Form vorliegen.

9. Zusammensetzung nach Anspruch 8, **dadurch gekennzeichnet, dass** das Silbersulfadiazin, das mindestens eine Silikat und/oder der mindestens eine pharmazeutisch annehmbare Exzipient in einer Teilchengröße von kleiner als 13 µm vorliegen, und/oder 90 Gew.-% der Teilchen eine Größe von zwischen 5 µm und 10 µm aufweisen and/oder 50 Gew.-% der Teilchen eine Größe von kleiner als 4 µm aufweisen.

10. Zusammensetzung nach einem der Ansprüche 1 - 9, **dadurch gekennzeichnet, dass** die Zusammensetzung kein Wasser und/oder organische Lösungsmittel enthält.

11. Zusammensetzung nach einem der Ansprüche 1 - 10 zur Verwendung bei der Behandlung und/oder Verhinderung einer Infektion bei Hautverletzungen.

12. Zusammensetzung nach einem der Ansprüche 1 - 11, **dadurch gekennzeichnet, dass** die Zusammensetzung von ein- bis drei-mal täglich verabreicht wird, vorzugsweise zweimal täglich.

## Revendications

1. Composition topique sous forme de pulvérisation de poudre, contenant de la sulfadiazine d'argent et au moins un silicate, **caractérisée en ce que** ladite composition contient une quantité de sulfadiazine d'argent comprise entre 0,1 % et 5 % en poids par rapport au poids total de la composition et une quantité d'au moins un silicate comprise entre 92 % et 99 % en poids par rapport au poids total de la composition.

2. Composition selon la revendication 1, **caractérisée en ce que** ladite composition contient une quantité de sulfadiazine d'argent comprise entre 0,5 % et 2 % en poids par rapport au poids total de la composition.

3. Composition selon l'une quelconque des revendications 1 à 2, **caractérisée en ce que** ladite composition contient une quantité d'au moins un silicate comprise entre 94 % et 98 % en poids du poids total de la composition.

4. Composition selon l'une quelconque des revendications précédentes, **caractérisée en ce que** ledit silicate est choisi parmi un silicate de magnésium hydraté, un silicate d'aluminium, un silicate de calcium ou un mélange de ceux-ci, de préférence un silicate d'aluminium.

5. Composition selon la revendication 3, **caractérisée en ce que** ledit silicate d'aluminium est un kaolin, de préférence un kaolin léger.

6. Composition selon l'une quelconque des revendications 1 à 3, **caractérisée en ce que** ladite composition contient au moins un excipient pharmaceutiquement acceptable.

7. Composition selon la revendication 6, **caractérisée en ce que** ledit au moins un excipient est choisi parmi le gluconate de calcium et/ou la silice colloïdale.

8. Composition selon l'une quelconque des revendications 1 à 7, **caractérisée en ce que** ladite sulfadiazine d'argent, ledit au moins un silicate et/ou ledit au moins un excipient pharmaceutiquement acceptable sont sous forme micronisée.

9. Composition selon la revendication 8, **caractérisée en ce que** ladite sulfadiazine d'argent, ledit au moins un silicate et/ou ledit au moins un excipient pharmaceutiquement acceptable présentent une taille de particule inférieure à 13 µm, et/ou 90 % des particules ont une taille comprise entre 5 µm et 10 µm et/ou 50 % des particules ont une taille inférieure à 4 µm.

10. Composition selon l'une quelconque des revendications 1 à 9, **caractérisée en ce que** ladite composition ne contient pas d'eau et/ou de solvants organiques.

11. Composition selon l'une quelconque des revendications 1 à 10 destinée à être utilisée dans le traitement et/ou la prévention d'infections de lésions cutanées.

12. Composition selon l'une quelconque des revendications 1 à 11, **caractérisée en ce que** ladite composition est administrée de une à trois fois par jour, de préférence deux fois par jour.
